(19) Europäisches Patentamt / European Patent Office / Office européen des brevets

(11) **EP 3 949 940 A1**

(12) **EUROPEAN PATENT APPLICATION**
published in accordance with Art. 153(4) EPC

(43) Date of publication:
**09.02.2022 Bulletin 2022/06**

(21) Application number: **20785000.9**

(22) Date of filing: **06.04.2020**

(51) International Patent Classification (IPC):
**A61K 8/03** (2006.01)    **A61K 8/19** (2006.01)
**A61K 8/25** (2006.01)    **A61K 8/34** (2006.01)
**A61K 8/41** (2006.01)    **A61K 8/72** (2006.01)
**A61K 8/81** (2006.01)    **A61Q 5/00** (2006.01)
**A61Q 5/06** (2006.01)

(52) Cooperative Patent Classification (CPC):
**A61K 8/03; A61K 8/19; A61K 8/25; A61K 8/34;
A61K 8/41; A61K 8/72; A61K 8/81; A61Q 5/00;
A61Q 5/06**

(86) International application number:
**PCT/JP2020/015485**

(87) International publication number:
**WO 2020/204206 (08.10.2020 Gazette 2020/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.04.2019 JP 2019072121
25.12.2019 JP 2019234986**

(71) Applicant: **Kao Corporation
Chuo-ku
Tokyo 103-8210 (JP)**

(72) Inventors:
• **SAITO, Yoichi
Tokyo 131-8501 (JP)**
• **NAKAOKA, Shiho
Tokyo 131-8501 (JP)**

(74) Representative: **Hoffmann Eitle
Patent- und Rechtsanwälte PartmbB
Arabellastraße 30
81925 München (DE)**

(54) **MULTILAYER TYPE COMPOSITION**

(57)    The present invention relates to a multilayer type composition containing the following components (A) to (D):
(A) an inorganic powder,
(B) a cationic polymer,
(C) an alkaline agent, and
(D) a nonaqueous solvent.

EP 3 949 940 A1

## Description

Field of the Invention

The present invention relates to a multilayer type composition.

Background of the Invention

A multilayer type composition composed of two layers or more layers is often configured of two layers of oil layer-water layer or liquid layer-powder layer. All of these are in a uniform milky lotion state or a powder-dispersed state by shaking, and when allowed to stand, the dispersed oils or powders gather in the upper part or the lower part and become a multilayer state again.

Even cosmetics in which various powders functioning as a colorant, a UV protection agent, a carrier, or the like are blended may take the form of a multilayer type composition composed of at least one liquid layer and a powder layer in a stationary state. However, in a multilayer type composition composed of a liquid layer and a powder layer, particularly in a multilayer type composition containing an inorganic powder having a high specific gravity as a whole, since the powders are liable to aggregate with each other, there is a case where a phenomenon (caking) in which the powders precipitate with a lapse of time and are solidified hard occurs, so that they are not redispersed even by shaking.

With respect to the improvement of such caking, PTL 1 discloses a sunscreen composition in which a trimellitic acid ester and a silicone-based surfactant are added to an inorganic powder having a UV protection effect, thereby improving dispersion stability of the inorganic powder. It is described that the foregoing sunscreen composition can be formed as a multilayer type composition such that it is readily uniformly mixed to become a cloudy emulsified state by shaking, and when allowed to stand, it is rapidly separated and returned to the original state.

In addition, as a hair cosmetic in which a tabular inorganic powder capable of imparting a pearl luster is blended, PTL 2 discloses a hair cosmetic containing (A) a cationic polymer and (B) a nonionic polymer in an amount of 0.5% by weight or more in terms of a total amount of the both such that a weight ratio of (A)/(B) is 1/2 to 8/1, and 0.0005 to 1.0% by weight of (C) micaceous titanium, and it is described that precipitation of the micaceous titanium can be prevented.

Citation List

Patent Literature

PTL 1: JP 2016-30725 A
PTL 2: JP 1-121209 A

Summary of the Invention

The present invention relates to the following.

[1] A multilayer type composition containing the following components (A) to (D):

(A) an inorganic powder,
(B) a cationic polymer,
(C) an alkaline agent, and
(D) a nonaqueous solvent.

[2] Use of, as a hair cosmetic, a multilayer type composition containing the following components (A) to (D):

(A) an inorganic powder,
(B) a cationic polymer,
(C) an alkaline agent, and
(D) a nonaqueous solvent.

Detailed Description of the Invention

[Multilayer Type Composition]

The multilayer type composition of the present invention contains the following components (A) to (D):

(A) an inorganic powder,
(B) a cationic polymer,
(C) an alkaline agent, and
(D) a nonaqueous solvent.

In view of the fact that the multilayer type composition of the present invention has the aforementioned constitution, there can be provided a composition in which even if the inorganic powder is contained, the occurrence of caking is less, and the redispersibility is favorable, and in the case of being used as a temporary hair dye, dyeing unevenness is suppressed, and drying after application is fast.

The multilayer type composition of the present invention is a composition in which though a powder including the component (A) is readily dispersed and homogenized by shaking, at least two layers of a liquid layer and a powder layer including the component (A) are formed within 12 hours after allowing to stand.

The hair cosmetic of PTL 2 is aimed at forming a film having an appropriate hardness on the hair, thereby imparting a strong setting effect. In addition, in the hair cosmetic described in PTL 2, the content of micaceous titanium is small as 0.0005 to 1.0% by weight, and in the working examples, even after preserving in a thermostat at 50°C for one month, the precipitation of micaceous titanium is prevented. In consequence, the hair cosmetic that is a disclosed invention of PTL 2 is perceived to be a single-layer type composition.

Now, among hair cosmetics, a temporary hair dye (e.g., a hair mascara) undergoes hair dyeing by forming a colored film including a colorant (mainly a pigment) on the hair, and therefore, so that the blending amount of the colorant is large, and it may take a form of a multilayer type composition depending upon the formulation. The temporary hair dye having a product formulation of mascara type can be used after shaking a mascara container by hand before application to undergo redispersion, so that it may take a form of a multilayer type composition composed of a liquid layer and a powder layer.

The temporary hair dye of such a form is required such that after preservation, the occurrence of caking of the colorant is less, and the redispersibility is favorable. However, among the colorants, an inorganic powder having a high specific gravity, such as a pearl pigment, is liable to be solidified hard when it precipitates and accumulates in the composition. When the caking of the colorant occurs, the concentration of the colorant which is dispersed in the temporary hair dye decreases, so that there is a concern that a hair dying effect is reduced, and dyeing unevenness due to aggregation of the colorant is caused.

Furthermore, as for required properties as the temporary hair dye, it is demanded that after applying on the hair, the temporary hair dye is rapidly dried, so that the colorant does not attach to the hand, etc.

The present invention relates to a multilayer type composition which contains an inorganic powder, is less in occurrence of caking even after preservation, and is favorable in redispersibility, and in which when used as the temporary hair dye, dyeing unevenness is suppressed, and drying after application is fast.

The present inventors have found that a multilayer type composition containing an inorganic powder, a cationic polymer, an alkaline agent, and a nonaqueous solvent satisfies the aforementioned requirements.

In accordance with the present invention, it is possible to provide a multilayer type composition which even when containing an inorganic powder, is less in occurrence of caking and favorable in redispersibility. The foregoing multilayer type composition is suitable for use as a hair cosmetic, particularly a temporary hair dye and is able to provide a temporary hair dye which is less in dyeing unevenness and fast in drying after application.

In the present invention, though the reason why the aforementioned effects are obtained is not elucidated yet, the following may be considered.

The inorganic powder that is the component (A) is liable to be solidified hard when it precipitates and accumulates in the multilayer type composition. In particular, an inorganic powder having a high specific gravity, such as a pearl pigment, tends to readily undergo hard caking. Here, when the multilayer type composition contains the cationic polymer that is the component (B) and the alkaline agent that is the component (C), the surface of the component (A), such as a pearl pigment, is negatively charged, and a bulky soft aggregate is formed and precipitated due to an electrostatic interaction between this surface and the cationic component (B). For that reason, it may be considered that the multilayer type composition can be readily redispersed after precipitation by shaking without causing hard caking.

When the multilayer type composition contains the nonaqueous solvent as the component (D), and furthermore, the content of water is preferably 70% by mass or less, aggregation of the component (A) is less, and in the case of being used as the temporary hair dye, not only the dyeing unevenness is suppressed, but also drying after application is fast, and handling properties at the time of use become favorable.

The multilayer type composition of the present invention is applicable to various uses and can be used as various cosmetics, such as a hair cosmetic, a sunscreen cosmetic, and a makeup cosmetic. Of these, the multilayer type composition of the present invention is more preferably a hair cosmetic from the viewpoint of efficacy of the effects of the present invention.

Examples of the hair cosmetic include a hair shampoo, a hair rinse, a hair conditioning agent, a hair treatment agent (inclusive of a non-rinsing-off type), a hair styling agent, a hair dye, and a hair growth promoter. Of these, the multilayer type composition of the present invention is preferably a hair dye, and more preferably a temporary hair dye from the viewpoint that the form of the multilayer type composition is readily applied.

<Component (A): Inorganic Powder>

The multilayer type composition of the present invention contains an inorganic powder as the component (A). Even when the multilayer type composition of the present invention contains the component (A) which is easy to cause the caking, it is able to suppress the occurrence of caking due to the aforementioned mechanism of action and is favorable in redispersibility. In the present invention, it should be construed that a powder in which a surface of an inorganic particle is coated with an organic material is also included in the component (A), and the foregoing powder may be constituted of an inorganic material to an extent of preferably 60% by mass or more, more preferably 70% by mass or more, and still more preferably 80% by mass or more.

Although any material can be used as the inorganic powder irrespective of the powder shape (e.g., a tabular shape, a spherical shape, and an acicular shape), from the viewpoint that the effects of the present invention are more efficiently obtained, the component (A) is more efficient in the case of including a tabular inorganic powder which is easy to cause the caking, and is still more efficient in the case of including a pearl pigment that is a tabular inorganic powder having a high specific gravity. Although the tabular inorganic powder having a high specific gravity readily causes the hard caking, according to the present invention, the occurrence of such caking can be effectively suppressed.

As the pearl pigment, all of an interference pearl pigment and a colored pearl pigment can be used, and these can also be used in combination.

The interference pearl pigment is a pigment exhibiting a pearly luster owing to a reflection and interference action of light and refers to one having a coating layer on a surface of a core particle, the coating layer including a reflection and interference layer and having translucency. The core particle is typically a semi-transparent tabular particle, and the reflection and interference layer is constituted of a colorless metal oxide, such as titanium dioxide, or a colorless metal. In addition, though the coating layer occasionally exhibits a wavelength color originated from its reflected light depending upon the thickness of the reflection and interference layer included therein, the coating layer per se has translucency.

In the present invention, the coating layer in the interference pearl pigment does not substantially contain a colored metal oxide and a colored metal. It is meant by the wording "does not substantially contain" that the coating layer does not contain any of a colored metal oxide and a colored metal within a range where the translucency of the coating layer per se does not appear. The content of the colored metal oxide and the colored metal in the coating layer is preferably 3% by mass or less, more preferably 1% by mass or less, and still more preferably 0.1% by mass or less. Examples of the colored metal oxide include iron oxide, copper oxide, cobalt oxide, chromium oxide, and nickel oxide, and examples of the colored metal include gold and copper.

A pigment having a coating layer on a surface of a core particle, the coating layer including a reflection and interference layer constituted of a colorless metal oxide or a colorless metal and a colored layer formed on a surface of the reflection and interference layer and constituted of an organic pigment is, in general, occasionally named as a composite pearl pigment. However, in the present invention, such a pigment is included in the interference pearl pigment so long as the coating layer has translucency as a whole.

Examples of a material of the core particle constituting the interference pearl pigment include mica, sericite, talc, kaolin, a smectite group clay mineral, bismuth oxychloride, synthetic mica, synthetic sericite, tabular silica, and tabular alumina. Of these, mica is preferred.

The reflection and interference layer included in the coating layer of the interference pearl pigment is preferably a layer formed of a thin film composed mainly of a colorless metal oxide, such as titanium dioxide, zinc oxide, and aluminum oxide, or a colorless metal, such as titanium, zirconium, zinc, tin, silicon, and aluminum, and it may be of a singlelayer structure or a multilayer structure. The colorless metal oxide or colorless metal constituting the reflection and interference layer is more preferably at least one selected from the group consisting of titanium and titanium dioxide, and still more preferably titanium dioxide.

The interference pearl pigment is more preferably titanium dioxide-coated mica (micaceous titanium).

Although a ratio between the core particle and the reflection and interference layer constituting the interference pearl pigment is not particularly restricted, a mass ratio of the core particle to the colorless metal oxide and the colorless metal constituting the reflection and interference layer [(core particle)/(colorless metal oxide and colorless metal constituting the reflection and interference layer)] is preferably 25/75 to 80/20, more preferably 30/70 to 70/30, and still more preferably 50/50 to 65/35.

The colored pearl pigment is a pigment exhibiting a pearly luster and has a coating layer on a surface of a core particle, and the foregoing coating layer is colored with the aforementioned colored metal oxide or colored metal and does not have translucency. In consequence, different from the interference pearl pigment, the colored pearl pigment has concealability. The core particle, the colored metal oxide, and the colored metal, which are used for the colored pearl pigment, are the same as those mentioned above.

Specific examples of the colored pearl pigment include iron oxide-coated mica, iron oxide-coated micaceous titanium, black iron oxide-coated mica, black iron oxide-coated micaceous titanium, yellow iron oxide-coated mica, iron oxide/black iron oxide-coated micaceous titanium, iron oxide/Prussian blue-coated micaceous titanium, iron oxide-coated bismuth oxychloride, and iron oxide-coated bismuth oxychloride mica.

It is more preferred that the component (A) includes the interference pearl pigment among the pearl pigments. In view of the fact that the component (A) includes the interference pearl pigment, when the multilayer type composition of the present invention is used as a temporary hair dye, particularly a temporary hair dye imparting a brilliant color to the hair for the purpose of improving the fashionability, a color-developing behavior is hardly influenced by a base hair color, so that a fixed hair dyeing effect can be imparted. With respect to the wording "color-developing behavior is hardly influenced by a base hair color, so that a fixed hair dyeing effect is imparted" as referred to herein, in detail, it is meant that regardless of whether the base hair color is a bright color or a dark color, a color change is produced according to the applied color without significantly changing the brightness (lightness) of the hair before and after hair dyeing.

In the case of a temporary hair dye aiming at improvement of the fashionability, though bright color development is preferred, the color development of a conventional temporary hair dye is readily influenced by the base hair color, and in the case of using a temporary hair dye having the same composition, a degree of color development is different depending upon whether the hair with a dark color, such as black hair, or the hair with a bright color, such as bleached hair, is to be dyed. In the case where it is intended to dye the hair with a dark color, such as black hair, a color change is hardly seen, and therefore, in order to obtain sufficient color development, it is needed to blend a considerable amount of a colorant, such as an organic pigment. However, when a temporary hair dye having a considerable amount of the colorant blended therein is applied on the hair with a bright color, such as a bleached hair, the color development is occasionally too gaudy.

Since the interference pearl pigment has translucency, even if the interference pearl pigment is applied alone on the hair with a bright color, the color change is hardly seen. However, when the interference pearl pigment is applied on the hair with a dark color, not only a glittering luster is provided owing to a reflection and interference action of light, but also the color change is readily seen. For that reason, when using the interference pearl pigment, even if a large amount of a colorant, such an organic pigment, is not blended, in the case of applying on the hair with a dark color, a temporary hair dye exhibiting sufficient color development is obtained, too. In addition, when applying the foregoing temporary hair dye on the hair with a bright color, desired color development can be imparted without significantly changing the brightness of hair. It may be considered that according to the foregoing mechanism of action, the color-developing behavior is hardly influenced by a base hair color, so that a fixed hair dyeing effect can be imparted.

Examples of the inorganic powder which is used as the component (A) other than pearl pigment include inorganic black pigments, such as carbon black, black iron oxide, and black titanium oxide; inorganic red pigments, such as iron oxide (red iron oxide), iron hydroxide, and iron titanate; inorganic brown pigments, such as $\gamma$-iron oxide; inorganic yellow pigments, such as yellow iron oxide and ocher; inorganic blue pigments, such as ultramarine blue and Prussian blue; inorganic violet pigments, such as manganese violet and cobalt violet; inorganic green pigments, such as chromium oxide, chromium hydroxide, and cobalt titanate; inorganic white pigments, such as titanium oxide, zinc oxide, cerium oxide, and barium sulfate; and metal powder pigments, such as gold powder, silver powder, copper powder, aluminum powder, and brass powder.

Although the specific gravity of the component (A) is not particularly limited, it is preferably 1.0 g/100 mL or more, more preferably 1.5 g/100 mL or more, still more preferably 3.0 g/100 mL or more, yet still more preferably 5.0 g/100 mL or more, and even yet still more preferably 10 g/100 mL or more in terms of a loose bulk specific gravity from the viewpoint that the effects of the present invention are more efficiently obtained. In the present invention, even if the component (A) is an inorganic powder having a high specific gravity as mentioned above, the occurrence of caking can be effectively suppressed. Although an upper limit of the loose bulk specific gravity of the component (A) is also not particularly restricted, it is preferably 40 g/100 mL or less, and more preferably 35 g/100 mL or less from the viewpoint of obtaining a multilayer type composition with favorable redispersibility. A specific range of the loose bulk specific gravity of the component (A) is preferably 1.0 to 40 g/100 mL, more preferably 1.5 to 40 g/100 mL, still more preferably 3.0 to 40 g/100 mL, yet still more preferably 5.0 to 40 g/100 mL, and even yet still more preferably 10 to 35 g/100 mL.

The loose bulk specific gravity of the component (A) can be measured in conformity with JIS K5101-12:2004 "Test methods for pigment -apparent density or apparent specific volume-".

Although an average particle diameter of the component (A) is not particularly limited, it is preferably 2 pm or more, more preferably 5 pm or more, and still more preferably 10 pm or more, and preferably 40 pm or less, more preferably 35 pm or less, and still more preferably 30 pm or less in terms of a volume median particle diameter (D50). A specific range of the volume median particle diameter (D50) of the component (A) is preferably 2 to 40 $\mu$m, more preferably 5 to 35 $\mu$m, and still more preferably 10 to 30 pm.

When the D50 of the component (A) falls within the aforementioned range, a multilayer type composition with favorable redispersibility is obtained. In addition, in the case where the component (A) is a colorant, such as a pearl pigment, the color developability is excellent, too.

The D50 of the component (A) is a value measured with a laser diffraction particle size analyzer.

As the component (A), a commercially available inorganic powder can be used. Examples of a commercially available interference pearl pigment include "Timiron Super Blue" (micaceous titanium, mica: 51% by mass, titanium dioxide: 49% by mass, D50: 18 to 25 $\mu$m, loose bulk specific gravity: 23 to 33 g/100 mL), "Timiron Super Gold" (micaceous titanium, mica: 60% by mass, titanium dioxide: 40% by mass, D50: 18 to 25 $\mu$m, loose bulk specific gravity: 16 to 26 g/100 mL), "Timiron Super Green" (micaceous titanium, mica: 55% by mass, titanium dioxide: 45% by mass, D50: 18 to 25 $\mu$m, loose bulk specific gravity: 20 to 30 g/100 mL), and "Timiron Super Red" (micaceous titanium, mica: 52% by mass, titanium dioxide: 48% by mass, D50: 18 to 25 $\mu$m, loose bulk specific gravity: 21 to 31 g/100 mL), all being available from Merck.

Examples of a commercially available colored pearl pigment which is used as the component (A) include "Timica Golden Bronze" (iron oxide-coated micaceous titanium, D50: 24 $\mu$m, loose bulk specific gravity: 3.3 g/100 mL) and "Cloisonne Super Green" (iron oxide/Prussian blue-coated micaceous titanium, D50: 21 $\mu$m), both being available from BASF; and "COLORONA BORDEAUX" (iron oxide-coated mica, D50: 18 to 25 $\mu$m, loose bulk specific gravity: 3 g/100 mL), "COLORONA LIGHT BLUE" (Prussian blue-coated micaceous titanium, D50: 18 to 25 $\mu$m, loose bulk specific gravity: 3 g/100 mL), "COLORONA Blackstar BLUE" (iron oxide-coated mica, D50: 18 to 25 $\mu$m, loose bulk specific gravity: 3 g/100 mL), "COLORONA FINE GOLD MP-20" (iron oxide-coated micaceous titanium, D50: 7 to 14 $\mu$m, loose bulk specific gravity: 17 to 27 g/100 mL), "COLORONA PRECIOUS GOLD" (metal oxide-coated micaceous titanium, mica: 35.5% by mass, titanium dioxide: 24% by mass, iron oxide: 20% by mass, silica: 20% by mass, tit oxide: 0.5% by mass, D50: 18 to 25 $\mu$m, loose bulk specific gravity: 3 g/100 mL), "COLORONA RED BROWN" (iron oxide-coated micaceous titanium, D50: 18 to 25 $\mu$m, loose bulk specific gravity: 17 to 27 g/100 mL), and "COLORONA DARK BLUE" (Prussian blue-coated micaceous titanium, D50: 18 to 25 $\mu$m, loose bulk specific gravity: 3 g/100 mL), all being available from Merck.

From the viewpoint of stably forming the powder layer in the multilayer type composition, the content of the component (A) in the multilayer type composition of the present invention is preferably 1.0% by mass or more, more preferably 2.0% by mass or more, still more preferably 3.0% by mass or more, yet still more preferably 3.5% by mass or more, even yet still more preferably 4.0% by mass or more, even still more preferably 5.0% by mass or more, and even still more further preferably 6.0% by mass or more. In addition, from the viewpoint of suppression of caking and redispersibility improving effect, the foregoing content is preferably 12.0% by mass or less, more preferably 11.0% by mass or less, still more preferably 10.0% by mass or less, and yet still more preferably 9.5% by mass or less. A specific range of the content of the component (A) in the multilayer type composition of the present invention is preferably 1.0 to 12.0% by mass, more preferably 2.0 to 12.0% by mass, still more preferably 3.0 to 11.0% by mass, 3.5 to 10.0% by mass, even yet still more preferably 4.0 to 10.0% by mass, even still more preferably 5.0 to 10.0% by mass, and even still more further preferably 6.0 to 9.5% by mass.

(Component (A)': Powder Other Than Component (A))

The multilayer type composition of the present invention may contain a powder other than the component (A) (hereinafter also referred to as "component (A)'") according to the use. The powder other than the component (A) is one which is an organic powder and is able to exist in a powder state without being dissolved in the multilayer type composition, and it is dispersed in the multilayer type composition or forms a powder layer together with the component (A).

As for the powder other than the component (A), any powder can be used irrespective of the powder shape (e.g., a tabular shape, a spherical shape, and an acicular shape), the particle diameter, and the particle structure (e.g., a porous structure and a nonporous structure).

In the case where the multilayer type composition is a temporary hair dye, from the viewpoint that hair dyeing properties of the component (A) are not affected, and in the case of being used as the temporary hair dye, a desired color shade can be imparted, the component (A)' is preferably an organic pigment. Examples of the organic pigment include Red No. 201, Red No. 202, Red No. 203, Red No. 204, Red No. 205, Red No. 206, Red No. 207, Red No. 208, Red No. 219, Red No. 220, Red No. 221, Red No. 226, Red No. 228, Red No. 404, Red No. 405, Orange No. 203, Orange No. 204, Orange No. 401, Yellow No. 205, Yellow No. 401, and Blue No. 404.

As the component (A)' other than those mentioned above, glittering powders, such as a polyethylene terephthalate/aluminum/epoxy laminate and a polyethylene terephthalate/polyolefin laminated film, can also be used.

The component (A)' can be used alone or in appropriate combination of two or more thereof according to the desired color shade.

In the case of using the component (A)', the content of the component (A)' in the multilayer type composition of the present invention is not particularly restricted and can be appropriately selected according to the use. The total content of the component (A) and the component (A)' in the multilayer type composition of the present invention is preferably 1.0% by mass or more, more preferably 2.0% by mass or more, still more preferably 3.0% by mass or more, yet still more preferably 3.5% by mass or more, even yet still more preferably 4.0% by mass or more, even still more preferably 5.0% by mass or more, and even still more further preferably 6.0% by mass or more from the viewpoint of stably forming the powder layer in the multilayer type composition. In addition, the foregoing total content is preferably 14.0% by mass or less, more preferably 13.0% by mass or less, and still more preferably 12.0% by mass or less from the viewpoint of suppression of caking and redispersibility improving effect. A specific range of the total content of the component (A) and the component (A)' in the multilayer type composition of the present invention is preferably 1.0 to 14.0% by mass, more preferably 2.0 to 13.0% by mass, still more preferably 2.0 to 12.0% by mass, yet still more preferably 3.0 to 12.0% by mass, even yet still more preferably 3.5 to 12.0% by mass, even still more preferably 4.0 to 12.0% by mass, even still more further preferably 5.0 to 12.0% by mass, and even yet still more further preferably 6.0 to 12.0% by mass.

<Component (B): Cationic Polymer>

The multilayer type composition of the present invention contains a cationic polymer as the component (B). In view of the fact that the multilayer type composition of the present invention contains the component (B) and an alkaline agent that is the component (C) as mentioned later, the occurrence of caking of the component (A) is suppressed, and the redispersibility becomes favorable due to the aforementioned mechanism of action.

In the present invention, the cationic polymer refers to a water-soluble polymer having a cation group or a group capable of being ionized to become a cation group (these groups will be hereinafter also referred to collectively as "cationic group"). The cationic polymer which is used in the present invention is preferably one having favorable dispersibility in a nonaqueous solvent that is the component (D) as mentioned later.

From the viewpoint of suppressing caking and improving redispersibility, a cation charge density of the component (B) is preferably 0.05 meq/g or more, more preferably 0.1 meq/g or more, still more preferably 0.2 meq/g or more, yet still more preferably 0.4 meq/g or more, and even yet still more preferably 0.5 meq/g or more, and preferably 6.5 meq/g or less, more preferably 3.8 meq/g or less, still more preferably 2.5 meq/g or less, yet still more preferably 2.0 meq/g or less, and even yet still more preferably 1.5 meq/g or less. A specific range of the cation charge density of the component (B) is preferably 0.05 to 6.5 meq/g, more preferably 0.1 to 3.8 meq/g, still more preferably 0.2 to 2.5 meq/g, yet still more preferably 0.4 to 2.0 meq/g, and even yet still more preferably 0.5 to 1.5 meq/g.

The cation charge density of the component (B) refers to [(molar number of the cationic group contained per 1 gram of the cationic polymer) $\times$ 1,000 (meq/g)].

As the component (B), the cationic polymer may be used in combination of two or more thereof, and it this case, the cation charge density of the cationic polymer is determined through calculation of a weighted average from the cation charge densities and the blending amounts of the respective cationic polymers.

Specific examples of the cationic polymer which is used as the component (B) include a cationized guar gum (e.g., Jaguar Excel (available from Solvay (Novecare)), a cationized tara gum (e.g., CATINAL CTR-100 (available from Toho Chemical Industry Co., Ltd.)), a cationized locust bean gum (e.g., CATINAL CLB-100 (available from Toho Chemical Industry Co., Ltd.)), a cationized polyvinyl alcohol (e.g., GOHSENX K-434 (available from The Nippon Synthetic Chemical Industry Co., Ltd.)), CM318 (available from Kuraray Co., Ltd.)), a vinylpyrrolidone/N,N-dimethylaminoethyl methacrylate copolymer diethyl sulfate (e.g., H·C POLYMER IS(M) and H·C POLYMER 2 (both being available from Osaka Organic Chemical Industry Ltd.)), a vinylpyrrolidone/dimethylaminopropyl methacrylamide/lauryl dimethylaminopropyl methacrylamide copolymer (e.g., Styleze W-20 (available from Ashland)), a dimethyldiallylammonium chloride/acrylamide copolymer (e.g., Marquat 550 (available from Lubrizol)), a vinylpyrrolidone/N,N-dimethylaminoethyl methacrylate copolymer diethyl sulfate (e.g., Gafquat 734 (available from Ashland)), a vinylpyrrolidone/N,N-dimethylaminoethyl methacrylate copolymer (e.g., Gafquat 440 (available from Ashland)), an N,N-dimethylaminoethyl methacrylate diethyl sulfate/N,N-dimethyl acrylamide/polyethylene glycol dimethacrylate copolymer (Polyquaternium-52) (e.g., Sofcare KG-101-E, Sofcare KG-101W-E, and Sofcare KG-301P (all being available from Kao Corporation)), ammonium-modified hydroxyethyl cellulose (e.g., a SoftCAT SL-30 polymer (available from The Dow Chemical Company)), an N-propionylpolyethyleneimine/methylpolysiloxane copolymer (polysilicone-9), and an acrylamide/DMAPA acrylate/methoxy methacrylate PEG copolymer. These can be used alone or in combination of two or more thereof.

Of these, from the viewpoint of dispersibility into a nonaqueous solvent that is the component (D) as mentioned later, the viewpoint of suppressing caking and improving redispersibility, and the viewpoint that in the case where the multilayer type composition is used as a temporary hair dye, color transfer after hair dyeing is less, and a feeling on the hair is improved, the component (B) is preferably at least one selected from the group consisting of a dimethyldiallylammonium chloride/acrylamide copolymer, a vinylpyrrolidone/N,N-dimethylaminoethyl methacrylate copolymer diethyl sulfate, an N,N-dimethylaminoethyl methacrylate diethyl sulfate/N,N-dimethyl acrylamide/polyethylene glycol dimethacrylate copolymer (Polyquaternium-52), ammonium-modified hydroxyethyl cellulose, an N-propionylpolyethyleneimine/methylpolysiloxane copolymer (polysilicone-9), and an acrylamide/DMAPA acrylate/methoxy methacrylate PEG copolymer; more preferably at least one selected from the group consisting of a dimethyldiallylammonium chloride/acrylamide copolymer, a vinylpyrrolidone/N,N-dimethylaminoethyl methacrylate copolymer diethyl sulfate, and an N,N-dimethylaminoethyl methacrylate diethyl sulfate/N,N-dimethyl acrylamide/polyethylene glycol dimethacrylate copolymer (Polyquaternium-52); and still more preferably an N,N-dimethylaminoethyl methacrylate diethyl sulfate/N,N-dimethyl acrylamide/polyethylene glycol dimethacrylate copolymer (Polyquaternium-52).

From the viewpoint of suppressing caking and improving redispersibility, the content of the component (B) in the multilayer type composition of the present invention is preferably 0.05% by mass or more, more preferably 0.1% by mass or more, and still more preferably 0.2% by mass or more, and preferably 5% by mass or less, more preferably 3% by mass or less, still more preferably 2% by mass or less, and yet still more preferably 0.8% by mass or less. A specific range of the content of the component (B) in the multilayer type composition is preferably 0.05 to 5% by mass, more preferably 0.1 to 3% by mass, still more preferably 0.1 to 2% by mass, and yet still more preferably 0.2 to 0.8% by mass.

<Component (C): Alkaline Agent>

The multilayer type composition of the present invention contains an alkaline agent as the component (C). In view of the fact that the multilayer type composition of the present invention contains the component (B) and the component (C), the occurrence of caking of the component (A) is suppressed, and the redispersibility becomes favorable due to the aforementioned mechanism of action. In addition, in the case of being used as the temporary hair dye, the hair dyeing properties can be improved.

As the component (C), alkaline agents which are typically used for cosmetics can be used without particular restrictions.

Examples of the alkaline agent include ammonia; alkanolamines, such as mono-, di-, or trimethanolamine, mono-, di-, or triethanolamine, and 2-amino-2-methyl-1-propanol; alkylamines, such as methylamine, dimethylamine, ethylamine, diethylamine, N-methylethylamine, propylamine, and butylamine; aralkylamines, such as benzylamine; and inorganic alkaline compounds, such as sodium hydroxide and potassium hydroxide. These can be used alone or in combination of two or more thereof. The carbon number of the alkanolamine, the alkylamine, or the aralkylamine is preferably 10 or less, and more preferably 8 or less from the viewpoint of water solubility.

Among those mentioned above, from the viewpoint of suppressing caking and improving redispersibility and the viewpoint of improving hair dyeing properties in the case of being used as the temporary hair dye, the component (C) preferably contains at least one selected from the group consisting of ammonia, an alkanolamine, an alkylamine, an aralkylamine, sodium hydroxide, and potassium hydroxide; more preferably contains at least one selected from the group consisting of an alkanolamine and sodium hydroxide; still more preferably at least one selected from the group consisting of a monoalkanolamine, 2-amino-2-methyl-1-propanol, and sodium hydroxide; and yet still more preferably at least one selected from the group consisting of monoethanolamine and sodium hydroxide.

From the viewpoint of suppressing caking and improving redispersibility, the content of the component (C) in the multilayer type composition of the present invention is preferably 0.01% by mass or more, more preferably 0.02% by mass or more, and still more preferably 0.05% by mass or more, and preferably 3% by mass or less, more preferably 2% by mass or less, still more preferably 1% by mass or less, and yet still more preferably 0.5% by mass or less. A specific range of the content of the component (C) in the multilayer type composition is preferably 0.01 to 3% by mass, more preferably 0.02 to 2% by mass, still more preferably 0.05 to 1% by mass, and yet still more preferably 0.05 to 0.5% by mass.

From the viewpoint of suppressing caking and improving redispersibility, a mass ratio [(B)/(C)] of the component (B) to the component (C) in the multilayer type composition of the present invention is preferably 0.1 or more, more preferably 0.5 or more, still more preferably 1 or more, and yet still more preferably 2 or more, and preferably 20 or less, more preferably 15 or less, still more preferably 10 or less, yet still more preferably 8 or less, and even yet still more preferably 5 or less. A specific range of the mass ratio [(B)/(C)] is preferably 0.1 to 20, more preferably 0.5 to 15, still more preferably 1 to 10, yet still more preferably 2 to 8, and even yet still more preferably 2 to 5.

<Component (D): Nonaqueous Solvent>

The multilayer type composition of the present invention contains, as the component (D), a nonaqueous solvent for the purpose of dispersing or dissolving the components (A) to (C) and other component. The nonaqueous solvent is a solvent other than water, and from the viewpoint of dispersing or dissolving the respective components in the multilayer type composition and the viewpoint of improving applicability and a drying rate after application in the case of being used as the temporary hair dye, a volatile polar solvent is preferably contained.

Examples of the volatile polar solvent include at least one selected from the group consisting of an alcohol, an ester, and a ketone, each having preferably 6 or less carbon atoms, and more preferably 4 or less carbon atoms, and it is preferred to contain an alcohol having 4 or less carbon atoms. Examples of the alcohol having 4 or less carbon atoms include ethanol, 1-propanol, 2-propanol (isopropanol), 1-butanol, and 2-butanol. Among the foregoing alcohols, at least one selected from the group consisting of ethanol and 2-propanol is more preferred, and from the viewpoint of improving a drying rate after application, ethanol is still more preferred.

The content of the at least one selected from the group consisting of ethanol and 2-propanol in the component (D) is preferably 30% by mass or more, more preferably 50% by mass or more, still more preferably 80% by mass or more, and yet still more preferably 85% by mass or more, and an upper limit thereof is 100% by mass from the viewpoint of dispersing or dissolving the respective components in the multilayer type composition and the viewpoint of improving applicability and a drying rate after application in the case of being used as the temporary hair dye. In addition, the content of ethanol in the component (D) is preferably 30% by mass or more, more preferably 50% by mass or more, and still more preferably 80% by mass or more, and an upper limit thereof is 100% by mass from the viewpoint of dispersing or dissolving the respective components in the multilayer type composition and the viewpoint of improving applicability and a drying rate after application in the case of being used as the temporary hair dye.

The content of the component (D) in the multilayer type composition of the present invention is preferably 30% by mass or more, more preferably 50% by mass or more, and still more preferably 70% by mass or more, and preferably 95% by mass or less from the viewpoint of dispersing or dissolving the respective components in the multilayer type composition and the viewpoint of improving applicability and a drying rate after application in the case of being used as the temporary hair dye.

In general, as compared to underwater, in the nonaqueous solvent, the electrostatic interaction between dispersed particles of the powder, such as the component (A), becomes weak, and a distance between the dispersed particles where electrostatic repulsion acts becomes short. For that reason, aggregation in a state where the interparticle distance is short to an extent that redispersion cannot be effected is liable to occur, so that the hard caking is readily achieved. However, even though the multilayer type composition of the present invention is not constituted of water as an essential component, it is possible to keep the redispersibility of the component (A) favorable. In addition, from the viewpoint that in the case of being used as the temporary hair dye, not only the dyeing unevenness is suppressed, but also drying after application is fast, and handling properties at the time of use become favorable, the content of water in the multilayer type composition is preferably 70% by mass or less, more preferably 40% by mass or less, still more preferably 30% by mass or less, yet still more preferably 20% by mass or less, even yet still more preferably 15% by mass or less, even still more preferably 10% by mass or less, even still more further preferably 5% by mass or less, even yet still more further preferably 2% by mass or less, and even yet still more further preferably 1% by mass or less, and a lower limit thereof is 0% by mass.

<Silicone Oil>

From the viewpoint of improving dispersibility of the component (B) and the viewpoint that in the case of being used as the temporary hair dye, a feeling on the hair after hair dyeing is improved, the multilayer type composition of the present invention can further contain a silicone oil.

As for the silicone oil, there are preferably exemplified a phenyl-modified silicone and a dimethyl silicone, each of which has a dynamic viscosity at 25°C of 200 mm$^2$/s or less.

Examples of the phenyl-modified silicone include methylphenyl polysiloxane (diphenyl dimethicone), phenyl methicone, and phenyl trimethicone. Specifically, examples thereof include KF-50-100cs (dynamic viscosity at 25°C: 100 mm$^2$/s), KF-53 (dynamic viscosity at 25°C: 175 mm$^2$/s), and KF-56 (dynamic viscosity at 25°C: 14 mm$^2$/s) (all being available from Shin-Etsu Chemical Co., Ltd.); and SH 556 FLUID (dynamic viscosity at 25°C: 14 mm$^2$/s) (available from Dow Corning Toray Co., Ltd.).

Examples of the dimethyl silicone include dimethyl polysiloxane (dimethicone). Specifically, examples thereof include KF-96A-200cs (dynamic viscosity at 25°C: 200 mm$^2$/s), KF-96A-100cs (dynamic viscosity at 25°C: 100 mm$^2$/s), KF-96A-50cs (dynamic viscosity at 25°C: 50 mm$^2$/s), KF-96A-30cs (dynamic viscosity at 25°C: 30 mm$^2$/s), KF-96A-20cs (dynamic viscosity at 25°C: 20 mm$^2$/s), KF-96A-10cs (dynamic viscosity at 25°C: 10 mm$^2$/s), KF-96A-6cs (dynamic viscosity at 25°C: 6 mm$^2$/s), KF-96A-5cs (dynamic viscosity at 25°C: 5 mm$^2$/s), KF-96L-2cs (dynamic viscosity at 25°C: 2 mm$^2$/s), KF-96L-1.5cs (dynamic viscosity at 25°C: 1.5 mm$^2$/s), and KF-96L-lcs (dynamic viscosity at 25°C: 1 mm$^2$/s) (all being available from Shin-Etsu Chemical Co., Ltd.); and SH200 C Fluid 200cs (dynamic viscosity at 25°C: 200 mm$^2$/s), SH200 C Fluid 100cs (dynamic viscosity at 25°C: 100 mm$^2$/s), SH200 C Fluid 50cs (dynamic viscosity at 25°C: 50 mm$^2$/s), SH200 C Fluid 30cs (dynamic viscosity at 25°C: 30 mm$^2$/s), SH200 C Fluid 20cs (dynamic viscosity at 25°C:

20 mm²/s), SH200 C Fluid IOcs (dynamic viscosity at 25°C: 10 mm²/s), SH200 C Fluid 6cs (dynamic viscosity at 25°C: 6 mm²/s), SH200 C Fluid 5cs (dynamic viscosity at 25°C: 5 mm²/s), SH200 C Fluid 2cs (dynamic viscosity at 25°C: 2 mm²/s), SH200 C Fluid 1.5cs (dynamic viscosity at 25°C: 1.5 mm²/s), and SH200 C Fluid 1cs (dynamic viscosity at 25°C: 1 mm²/s) (all being available from Dow Corning Toray Co., Ltd.).

From the viewpoint of improving dispersibility of the component (B) and the viewpoint that in the case where the multilayer type composition is used as the temporary hair dye, a feeling on the hair after hair dyeing is improved, the dynamic viscosity at 25°C of the silicone oil is preferably 200 mm²/s or less, more preferably 150 mm²/s or less, and still more preferably 100 mm²/s or less. The dynamic viscosity can be measured at 25°C with an Ubbelohde viscometer on the basis of JIS Z8803: 2011 "Methods for viscosity measurement of liquid".

The silicone oil can be used alone or in combination of two or more thereof.

From the viewpoint of improving dispersibility of the component (B) and the viewpoint that in the case where the multilayer type composition is used as the temporary hair dye, a feeling on the hair after hair dyeing is improved, the content of the silicone oil in the multilayer type composition is preferably 0.1% by mass or more, more preferably 0.5% by mass or more, and still more preferably 1% by mass or more, and preferably 20% by mass or less, more preferably 15% by mass or less, and still more preferably 10% by mass or less. A specific range of the content of the silicone oil in the multilayer type composition is preferably 0.1 to 20% by mass, more preferably 0.5 to 15% by mass, and still more preferably 1 to 10% by mass.

<Other Component>

To the multilayer type composition of the present invention, a component which is typically used for the cosmetic can be added in addition to the aforementioned components. Examples of the foregoing component include a surfactant; an oil, such as oils and fats and a sparingly volatile hydrocarbon; a thickener; a propellant, such as LPG (liquefied petroleum gas), pentane, dimethyl ether, and diethyl ether; and others, such as a perfume, an antiseptic, an ultraviolet absorber, a chelating agent, an antioxidant, and a vegetable extract.

A production method of the multilayer type composition of the present invention is not particularly limited, and the multilayer type composition can be produced by stirring and mixing the aforementioned respective components by using a known device.

<Formulation and Product Form>

The formulation of the multilayer type composition of the present invention is not particularly restricted so long as it is in a form that though the powder including the component (A) is readily dispersed and homogenized by shaking, two layers of at least the liquid layer and the powder layer including the component (A) are formed in a stationary state. For example, in the case of being as the temporary hair dye, its formulation is preferably a liquid having a viscosity range as mentioned later from the viewpoint of applying the temporary hair dye to a target site of the hair by using an application tool, such as a brush, a comb, and a sponge.

The product form of the temporary hair dye is preferably of a mascara type. When the product form is of a mascara type, the temporary hair dye can be easily applied in the target site of the hair by using an application tool, such as a brush and a sponge. In addition, the mascara-type temporary hair dye can be used through redispersion the multilayer type composition by shaking a mascara container by hand prior to application, and it is excellent in handling properties and suitable from the standpoint of portability.

In the case where the multilayer type composition is a temporary hair dye, its viscosity at 25°C may be more than 0 mPa·s from the viewpoint of minimizing liquid dripping during application, and the foregoing viscosity at 25°C is preferably 1 mPa·s or more, more preferably 5 mPa·s or more, and still more preferably 10 mPa·s or more from the viewpoint of suppressing liquid dripping and splash of liquid during the application operation, and it is preferably 3,000 mPa·s or less, more preferably 2,000 mPa·s or less, still more preferably 1,500 mPa·s or less, yet still more preferably 1,000 mPa·s or less, even yet still more preferably 800 mPa·s or less, even still more preferably 500 mPa·s or less, even still further preferably 300 mPa·s or less, and even yet still more further preferably 150 mPa·s or less from the viewpoint of increasing the holding amount on an applied body of the application tool and evenly applying in a single operation from the root to the tip of the hair without causing liquid outage.

The aforementioned viscosity is a value when measured at a temperature of 25±1°C for 1 minute at a predetermined rotation rate with a B-type viscometer using a rotor according to the measuring adaptive range. Specifically, 70 to 80 g of the temporary hair dye is charged in a screw tube having a capacity of 100 to 200 mL and shaken by hand 100 times in a width of about 30 cm for 30 seconds such that it becomes uniform; further, immediately after standing up 30 times at a speed of one time per second, using a B-type viscometer (TV-10M, available from Toki Sangyo Co., Ltd.), the viscosity is measured under conditions of using a rotor No. M1 for up to viscosities of 100 mPa·s at a rotation speed of 100 rpm, using a rotor No. M2 in a viscosity range of 100 to 500 mPa·s at a rotation speed of 60 rpm, using a rotor

**EP 3 949 940 A1**

No. M3 in a viscosity range of 500 to 2,000 mPa·s at a rotation speed of 60 rpm, and using a rotor No. M4 in a viscosity range of 2,000 to 10,000 mPa·s at a rotation speed of 60 rpm.

From the viewpoint of storage stability, a pH at 25°C of the multilayer type composition of the present invention is preferably 5.0 or more, more preferably 6.0 or more, still more preferably 7.0 or more, and yet still more preferably 8.0 or more, and preferably 12.0 or less, more preferably 11.5 or less, still more preferably 10.5 or less, and yet still more preferably 10.0 or less in terms of a pH as measured by diluting the composition with water 10 times. A specific range of the pH is preferably 5.0 to 12.0, more preferably 6.0 to 11.5, still more preferably 7.0 to 10.5, and yet still more preferably 8.0 to 10.0.

[Use]

The present invention also provides use of, a hair cosmetic, a multilayer type composition containing the following components (A) to (D):

(A) an inorganic powder,
(B) a cationic polymer,
(C) an alkaline agent, and
(D) a nonaqueous solvent.

The foregoing hair cosmetic is preferably a temporary hair dye. In addition, the content of water in the multilayer type composition is preferably 70% by mass or less.

The components contained in the aforementioned composition and preferred embodiments thereof are the same as those in the aforementioned multilayer type composition. For example, in the case where the aforementioned hair cosmetic is a temporary hair dye, as for the use method thereof, after applying the temporary hair dye on the hair through application, etc., it is allowed to stand and used without rinsing away.

With respect to the aforementioned embodiments, the present invention discloses the following multilayer type composition, use of the multilayer type composition as a hair cosmetic, and hair dyeing method.

<1> A multilayer type composition containing the following components (A) to (D):

(A) an inorganic powder,
(B) a cationic polymer,
(C) an alkaline agent, and
(D) a nonaqueous solvent.

<2> The multilayer type composition as set forth in <1>, wherein the content of water is preferably 70% by mass or less, more preferably 40% by mass or less, still more preferably 30% by mass or less, yet still more preferably 20% by mass or less, even yet still more preferably 15% by mass or less, even still more preferably 10% by mass or less, even still more further preferably 5% by mass or less, even yet still more further preferably 2% by mass or less, and even yet still more further preferably 1% by mass or less.

<3> A multilayer type composition containing the following components (A) to (D):

(A) an inorganic powder,
(B) a cationic polymer,
(C) an alkaline agent, and
(D) a nonaqueous solvent,

wherein a mass ratio [(B)/(C)] of the component (B) to the component (C) is 2 or more and 5 or less, and the content of water is 10% by mass or less.

<4> A multilayer type composition containing the following components (A) to (D):

(A) an inorganic powder,
(B) a cationic polymer,
(C) an alkaline agent, and
(D) a nonaqueous solvent,

wherein the component (D) contains 30% by mass or more of ethanol, a mass ratio [(B)/(C)] of the component (B) to the component (C) is 2 or more and 5 or less, and the content of water is 10% by mass or less.

<5> The multilayer type composition as set forth in any one of <1> to <4>, wherein the component (A) includes a tabular inorganic powder, preferably a pearl pigment, and more preferably an interference pearl pigment.

<6> The multilayer type composition as set forth in any one of <1> to <5>, wherein a cation charge density of the component (B) is preferably 0.05 meq/g or more, more preferably 0.1 meq/g or more, still more preferably 0.2 meq/g or more, yet still more preferably 0.4 meq/g or more, and even yet still more preferably 0.5 meq/g or more, and preferably 6.5 meq/g or less, more preferably 3.8 meq/g or less, still more preferably 2.5 meq/g or less, yet still more preferably 2.0 meq/g or less, and even yet still more preferably 1.5 meq/g or less.

<7> The multilayer type composition as set forth in any one of <1> to <6>, wherein the component (C) includes at least one selected from the group consisting of ammonia, an alkanolamine, an alkylamine, an aralkylamine, sodium hydroxide, and potassium hydroxide; preferably at least one selected from the group consisting of an alkanolamine and sodium hydroxide; more preferably at least one selected from the group consisting of a monoalkanolamine and 2-amino-2-methyl-1-propanol; and still more preferably monoethanolamine.

<8> The multilayer type composition as set forth in any one of <1> to <7>, wherein the component (D) is a volatile polar solvent and preferably at least one selected from an alcohol, an ester, and a ketone, each having 6 or less carbon atoms, and more preferably 4 or less carbon atoms.

<9> The multilayer type composition as set forth in any one of <1> to <8>, wherein the component (D) contains an alcohol having 4 or less carbon atoms, preferably at least one selected from the group consisting of ethanol, 1-propanol, 2-propanol, 1-butanol, and 2-butanol, more preferably at least one selected from the group consisting of ethanol and 2-propanol, and still more preferably ethanol.

<10> The multilayer type composition as set forth in any one of <1> to <9>, wherein the content of at least one selected from the group consisting of ethanol and 2-propanol in the component (D) is preferably 30% by mass or more, more preferably 50% by mass or more, still more preferably 80% by mass or more, and yet still more preferably 85% by mass or more, and an upper limit thereof is 100% by mass.

<11> The multilayer type composition as set forth in any one of <1> to <7>, wherein the component (D) contains 30% by mass or more, preferably 50% by mass or more, and more preferably 80% by mass or more of ethanol.

<12> The multilayer type composition as set forth in any one of <1> to <11>, wherein a loose bulk specific gravity of the component (A) is preferably 1.0 g/100 mL or more, more preferably 1.5 g/100 mL or more, still more preferably 3.0 g/100 mL or more, yet still more preferably 5.0 g/100 mL or more, and even yet still more preferably 10 g/100 mL or more, and preferably 40 g/100 mL or less, and more preferably 35 g/100 mL or less.

<13> The multilayer type composition as set forth in any one of <1> to <12>, wherein the content of the component (A) is preferably 1.0% by mass or more, more preferably 2.0% by mass or more, still more preferably 3.0% by mass or more, yet still more preferably 3.5% by mass or more, even yet still more preferably 4.0% by mass or more, even still more preferably 5.0% by mass or more, and even still more further preferably 6.0% by mass or more, and preferably 12.0% by mass or less, more preferably 11.0% by mass or less, still more preferably 10.0% by mass or less, and yet still more preferably 9.5% by mass or less.

<14> The multilayer type composition as set forth in any one of <1> to <13>, further containing an organic powder, preferably an organic pigment as a component (A)' that is a powder other than the component (A).

<15> The multilayer type composition as set forth in <14>, wherein the total content of the component (A) and the component (A)' is preferably 1.0% by mass or more, more preferably 2.0% by mass or more, still more preferably 3.0% by mass or more, yet still more preferably 3.5% by mass or more, even yet still more preferably 4.0% by mass or more, even still more preferably 5.0% by mass or more, and even still more further preferably 6.0% by mass or more, and preferably 14.0% by mass or less, more preferably 13.0% by mass or less, and still more preferably 12.0% by mass or less.

<16> The multilayer type composition as set forth in any one of <1> to <15>, wherein the component (B) is one, two or more selected from the group consisting of a cationized guar gum, a cationized tara gum, a cationized locust bean gum, a cationized polyvinyl alcohol, a vinylpyrrolidone/N,N-dimethylaminoethyl methacrylate copolymer diethyl sulfate, a vinylpyrrolidone/dimethylaminopropyl methacrylamide/lauryl dimethylaminopropyl methacrylamide copolymer, a dimethyldiallylammonium chloride/acrylamide copolymer, a vinylpyrrolidone/N,N-dimethylaminoethyl methacrylate copolymer diethyl sulfate, a vinylpyrrolidone/N,N-dimethylaminoethyl methacrylate copolymer, an N,N-dimethylaminoethyl methacrylate diethyl sulfate/N,N-dimethyl acrylamide/polyethylene glycol dimethacrylate copolymer, ammonium-modified hydroxyethyl cellulose, an N-propionylpolyethyleneimine/methylpolysiloxane copolymer, and an acrylamide/DMAPA acrylate/methoxy methacrylate PEG copolymer; preferably at least one selected from the group consisting of a dimethyldiallylammonium chloride/acrylamide copolymer, a vinylpyrrolidone/N,N-dimethylaminoethyl methacrylate copolymer diethyl sulfate, an N,N-dimethylaminoethyl methacrylate diethyl sulfate/N,N-dimethyl acrylamide/polyethylene glycol dimethacrylate copolymer, ammonium-modified hydroxyethyl cellulose, an N-propionylpolyethyleneimine/methylpolysiloxane copolymer, and an acrylamide/DMAPA acrylate/methoxy methacrylate PEG copolymer; more preferably at least one selected from the group consisting of a dimethyldiallylammonium chloride/acrylamide copolymer, a vinylpyrrolidone/N,N-dimethylaminoethyl methacrylate copolymer diethyl sulfate, and an N,N-dimethylaminoethyl methacrylate diethyl sulfate/N,N-dimethyl acrylamide/polyethylene glycol dimethacrylate copolymer; and still more preferably an N,N-dimethylaminoethyl methacrylate diethyl sulfate/N,N-dimethyl acrylamide/polyethylene glycol dimethacrylate copolymer.

<17> The multilayer type composition as set forth in any one of <1> to <16>, wherein the content of the component (B) is preferably 0.05% by mass or more, more preferably 0.1% by mass or more, and still more preferably 0.2% by mass or more, and preferably 5% by mass or less, more preferably 3% by mass or less, still more preferably 2% by mass or less, and yet still more preferably 0.8% by mass or less.

<18> The multilayer type composition as set forth in any one of <1> to <17>, wherein the content of the component (C) is preferably 0.01% by mass or more, more preferably 0.02% by mass or more, and still more preferably 0.05% by mass or more, and preferably 3% by mass or less, more preferably 2% by mass or less, still more preferably 1% by mass or less, and yet still more preferably 0.5% by mass or less.

<19> The multilayer type composition as set forth in any one of <1> to <18>, wherein the mass ratio [(B)/(C)] of the component (B) to the component (C) is preferably 0.1 or more, more preferably 0.5 or more, still more preferably 1 or more, and yet still more preferably 2 or more, and preferably 20 or less, more preferably 15 or less, still more preferably 10 or less, yet still more preferably 8 or less, and even yet still more preferably 5 or less.

<20> The multilayer type composition as set forth in any one of <1> to <19>, wherein the content of the component (D) is preferably 30% by mass or more, more preferably 50% by mass or more, and still more preferably 70% by mass or more, and preferably 95% by mass or less.

<21> The multilayer type composition as set forth in any one of <1> to <20>, further containing a silicone oil in an amount of preferably 0.1% by mass or more, more preferably 0.5% by mass or more, and still more preferably 1% by mass or more, and preferably 20% by mass or less, more preferably 15% by mass or less, and still more preferably 10% by mass or less.

<22> The multilayer type composition as set forth in any one of <1> to <21>, wherein a pH at 25°C of the multilayer type composition is preferably 5.0 to 12.0, more preferably 6.0 to 11.5, still more preferably 7.0 to 10.5, and yet still more preferably 8.0 to 10.0 in terms of a pH as measured by diluting the composition with water 10 times.

<23> The multilayer type composition as set forth in any one of <1> to <22>, which is a hair cosmetic.

<24> The multilayer type composition as set forth in <23>, wherein the hair cosmetic is a temporary hair dye.

<25> Use of, as a hair cosmetic, a multilayer type composition containing the following components (A) to (D):

    (A) an inorganic powder,
    (B) a cationic polymer,
    (C) an alkaline agent, and
    (D) a nonaqueous solvent.

<26> A hair dyeing method including applying hair with a multilayer type composition containing the following components (A) to (D):

    (A) an inorganic powder,
    (B) a cationic polymer,
    (C) an alkaline agent, and
    (D) a nonaqueous solvent.

<27> The use as set forth in <25> or the hair dyeing method as set forth in <26>, wherein the content of water in the multilayer type composition is preferably 70% by mass or less, more preferably 40% by mass or less, still more preferably 30% by mass or less, yet still more preferably 20% by mass or less, even yet still more preferably 15% by mass or less, even still more preferably 10% by mass or less, even still more further preferably 5% by mass or less, even yet still more further preferably 2% by mass or less, and even yet still more further preferably 1% by mass or less.

<28> The use as set forth in <25> or the hair dyeing method as set forth in <26>, wherein a pH at 25°C of the multilayer type composition is preferably 5.0 to 12.0, more preferably 6.0 to 11.5, still more preferably 7.0 to 10.5, and yet still more preferably 8.0 to 10.0 in terms of a pH as measured by diluting the composition with water 10 times.

Examples

The present invention is hereunder described by reference to Examples, but it should be construed that the present invention is not limited to the scope of the Examples.

Examples 1 to 6 and Comparative Examples 1 to 5 (Preparation and Evaluation of Temporary Hair Dye)

A mixture of the component (A) and the powder other than the component (A) (component (A)') as shown in Table 1 was added to the component (D) or water, followed by stirring with a disper disperser at 1,000 rpm for 5 minutes. To the resultant, the remaining component(s) was added and then stirred with a disper disperser at 1,000 rpm for 5 minutes, to obtain a temporary hair dye of each of the Examples, which is the multilayer type composition. The pH at 25°C of the temporary hair dye (value obtained by diluting with water 10 times and measuring with a pH meter (model: F-51), manufactured by HORIBA, Ltd.) was in a range of 8.5 to 9.5.

Using the obtained temporary hair dye, various evaluations were performed by the following methods.

<Storage Stability>

(Presence or Absence of Caking After Preservation)

The temporary hair dye obtained in each of the Examples was stirred with a disper disperser at 1,000 rpm for 5 minutes, 70 g of which was then taken into a transparent closed container (screw tube bottle, No. 8, transparent, available from Maruemu Corporation) in a state that the component (A) and the component (A)' were thoroughly dispersed, followed by hermetically sealing. After allowing to stand at 25°C for 12 hours, the closed container was inclined to the horizontal direction, and the presence or absence of caking was visually observed and evaluated according to the following evaluation criteria. An average value of the evaluation scores (rounded off from the first decimal place) by five evaluators is shown in Table 1.

[Evaluation Criteria and Score]

1: At least a part of the precipitate has fluidity, and the caking does not occur.
2: The precipitate does not have fluidity, and the caking occurs.

(Redispersibility)

The temporary hair dye obtained in each of the Examples was stirred with a disper disperser at 1,000 rpm for 5 minutes, 70 g of which was then taken into a transparent closed container (screw tube bottle, No. 8, transparent, available from Maruemu Corporation) in a state that the component (A) and the component (A)' were thoroughly dispersed, followed by hermetically sealing. After allowing to stand at 25°C for 12 hours, the closed container was shaken at a stroke of 30 cm one time per sec, and the dispersion state of the pigment, such as the component (A), was visually observed and evaluated according to the following evaluation criteria. An average value of the evaluation scores (rounded off from the first decimal place) by five expert panelists is shown in Table 1.

[Evaluation Criteria and Score]

1: The components are dispersed by shaking of not more than 5 times, and no aggregation is observed.
2: The components are dispersed by shaking of 6 times or more and 10 times or less, and no aggregation is observed.
3: Even after shaking of 11 times or more, some of the components keep the aggregated state.
4: Even after shaking of 11 times or more, the components are not dispersed.

<Hair Dyeing Properties>

(Color Difference ΔE* of Bleached Tress)

A bleached tress prepared by bleaching a black hair tress having a length of 10 cm and a mass of 1 g (BS-B-A, manufactured by Beaulax Co., Ltd.) into 9 tones (in a range where the lightness L* is 26.9 to 30.4, and the chroma C* is 18.0 to 20.6) was prepared. This tress was washed once with a plain hair shampoo having the following composition, air-dried, and then uniformly applied with 0.2 g of the temporary hair dye obtained in each of the Examples to perform hair dyeing, followed by allowing to stand for natural drying at 25°C for 30 minutes.

The tress before hair dyeing and after the aforementioned hair dyeing was measured in the CIE standard colorimetric system (L*,a*,b*) by using a color-difference meter (CR-400, available from Konica Minolta, Inc.), and a color difference ∆E* was calculated according to the following equation. The measurement of L*,a*,b* was performed at three different points on the tress (every one point in the center of each of three areas resulting through trisection of the tress in the lengthwise direction), and an average value thereof was calculated.

So long as in the bleached hair tress, ∆E* is 10 or more, the color change after hair dyeing can be actually felt, and the hair dyeing properties are favorable.

$$\Delta E^* = \sqrt{(L_1^* - L_0^*)^2 + (a_1^* - a_0^*)^2 + (b_1^* - b_0^*)^2}$$

$L_0^*$, $a_0^*$, $b_0^*$: measured value of the tress before hair dyeing
$L_1^*$, $a_1^*$, $b_1^*$: measured value of the tress after hair dyeing

[Composition of Plain Shampoo]

| Component | (% by mass) |
| --- | --- |
| Sodium polyoxyethylene(2) lauryl ether sulfate (*1) | 15.5 |
| Lauric acid diethanolamide (*2) | 1.5 |
| Tetrasodium edetate | 0.3 |
| Sodium benzoate | 1.43 |
| Purified water | Balance |
| Total | 100.0 |

*1: 42.0% by mass as EMAL 227 (available from Kao Corporation, active ingredient: 27% by mass)
*2: AMINON L-02 (available from Kao Corporation)

(Dyeing Unevenness of Bleached Tress)

With respect to the aforementioned bleached tress after hair dyeing, finishing was visually observed and evaluated according to the following evaluation criteria. An average value of the evaluation scores (rounded off from the first decimal place) by five expert panelists is shown in Table 1.

[Evaluation Criteria and Score]

1: Dyeing unevenness is not observed.
2: Dyeing unevenness is slightly observed, but it is within the tolerance.
3: Dyeing unevenness is observed.

<Drying Rate>

By applying a black hair tress having a length of 10 cm and a mass of 1 g (BS-B-A, manufactured by Beaulax Co., Ltd.) with 0.2 g of the temporary hair dye obtained in each of the Examples, allowing to stand at 25°C, and pressing against Kimwipes every one minute by using a 100-g weight, a time until soaking of the temporary hair dye into the Kimwipes was confirmed was evaluated.

Table 1

| | | Example | | | | | | Comparative Example | | | | |
|---|---|---|---|---|---|---|---|---|---|---|---|---|
| | | 1 | 2 | 3 | 4 | 5 | 6 | 1 | 2 | 3 | 4 | 5 |
| (A) | Micaceous titanium A *1 | 6.5 | 6.5 | 6.5 | 6.5 | 6.5 | 6.5 | 6.5 | 6.5 | 6.5 | 6.5 | 6.5 |
| Component (A)' other than component (A) | Yellow No. 401/barium sulfate *2 | 0.63 | 0.63 | 0.63 | 0.63 | 0.63 | 0.63 | 0.63 | 0.63 | 0.63 | 0.63 | 0.63 |
| | Red No. 226 *3 | 0.95 | 0.95 | 0.95 | 0.95 | 0.95 | 0.95 | 0.95 | 0.95 | 0.95 | 0.95 | 0.95 |
| | Blue No. 404 *4 | 0.95 | 0.95 | 0.95 | 0.95 | 0.95 | 0.95 | 0.95 | 0.95 | 0.95 | 0.95 | 0.95 |
| (B) | Polyquaternium-52 *5 | 0.3 | 0.3 | 0.3 | 0.3 | 0.15 | 1 | | | | 0.3 | 0.3 |
| Polymer other than component (B) | Carboxyvinyl polymer *6 | | | | | | | | | 0.3 | | |
| | Hydroxyethyl cellulose *7 | | | | | | | | | | 0.3 | |
| (C) | Monoethanolamine | 0.1 | 0.1 | 0.1 | | 0.1 | 0.1 | 0.1 | 0.1 | 0.1 | | 0.1 |
| | Sodium hydroxide | | | | 0.1 | | | | | | | |
| (D) | Ethanol | 85.6 | | 70.6 | 85.6 | 85.7 | 84.9 | 85.9 | 85.6 | 85.6 | 85.7 | |
| | Isopropanol | | 85.6 | | | | | | | | | |
| Methylphenyl polysiloxane *8 | | 5 | 5 | 5 | 5 | 5 | 5 | 5 | 5 | 5 | 5 | 5 |
| Water | | | | 15.0 | | | | | | | | 85.6 |
| Total | | 100.0 | 100.0 | 100.0 | 100.0 | 100.0 | 100.0 | 100.0 | 100.0 | 100.0 | 100.0 | 100.0 |
| Content of component (A) (% by mass) | | 6.5 | 6.5 | 6.5 | 6.5 | 6.5 | 6.5 | 6.5 | 6.5 | 6.5 | 6.5 | 6.5 |
| Total content of component (A) and component (A)' (% by mass) | | 9.0 | 9.0 | 9.0 | 9.0 | 9.0 | 9.0 | 9.0 | 9.0 | 9.0 | 9.0 | 9.0 |
| Content of component (B) (% by mass) | | 0.3 | 0.3 | 0.3 | 0.3 | 0.15 | 1 | 0 | 0 | 0 | 0.3 | 0.3 |
| Content of component (C) (% by mass) | | 0.1 | 0.1 | 0.1 | 0.1 | 0.1 | 0.1 | 0.1 | 0.1 | 0.1 | 0.0 | 0.1 |
| Content of component (D) (% by mass) | | 85.6 | 85.6 | 70.6 | 85.6 | 85.7 | 84.9 | 85.9 | 85.6 | 85.6 | 85.7 | 0.0 |
| Mass ratio (B)/(C) | | 3.0 | 3.0 | 3.0 | 3.0 | 1.5 | 10.0 | 0.0 | 0.0 | 0.0 | - | 3.0 |

| | | | Example | | | | | | Comparative Example | | | | |
|---|---|---|---|---|---|---|---|---|---|---|---|---|---|
| | | | 1 | 2 | 3 | 4 | 5 | 6 | 1 | 2 | 3 | 4 | 5 |
| Evaluation results | Storage stability | Presence or absence of caking after preservation | 1 | 1 | 1 | 1 | 1 | 1 | 2 | 2 | 2 | 2 | 1 |
| | | Redispersibility | 1 | 1 | 1 | 1 | 2 | 3 | 4 | 4 | 4 | 4 | 3 |
| | Hair dyeing properties | Bleached tress $\Delta E^*$ | 18.4 | 20.0 | 17.6 | 19.1 | 17.7 | 18.4 | 8.3 | 8.5 | 9.6 | 8.8 | 21.5 |
| | | Dyeing unevenness | 1 | 1 | 2 | 1 | 1 | 1 | 1 | 1 | 1 | 1 | 3 |
| | Drying rate (min) | | 1 | 2 | 2 | 1 | 1 | 1 | 1 | 1 | 1 | 1 | 5 |

The components described in Table 1 are shown below. In addition, the blending amounts of the components shown in Table 1 are each an effective amount (% by mass).

*1: Timiron Super Blue (available from Merck)
*2: 70% Yellow No. 401 barium sulfate (available from Kishi Kasei Co., Ltd.)
*3: D&C RED No. 30 (available from Daito Kasei Kogyo Co., Ltd.)
*4: Blue No. 404 (color of law) (available from Kishi Kasei Co., Ltd.)
*5: Sofcare KG-101-E (available from Kao Corporation, cation charge density: 0.94 meq/g)
*6: Carbopol 981 Polymer (available from Nippon Lubrizol Co., Ltd.; anionic polymer)
*7: HEC DAICEL SE850K (available from Daicel Corporation; nonionic polymer)
*8: Silicone SH 556 FLUID (available from Dow Corning Toray Co., Ltd.)

Industrial Applicability

In accordance with the present invention, it is possible to provide a multilayer type composition which even when containing an inorganic powder, is less in occurrence of caking and favorable in redispersibility. The foregoing multilayer type composition is suitable for use as a hair cosmetic, particularly a temporary hair dye and is able to provide a temporary hair dye which is less in dyeing unevenness and fast in drying after application.

**Claims**

1. A multilayer type composition comprising the following components (A) to (D):

    (A) an inorganic powder,
    (B) a cationic polymer,
    (C) an alkaline agent, and
    (D) a nonaqueous solvent.

2. The multilayer type composition according to claim 1, wherein the content of water is 70% by mass or less.

3. The multilayer type composition according to claim 1 or 2, wherein the component (A) includes a pearl pigment.

4. The multilayer type composition according to any one of claims 1 to 3, wherein a cation charge density of the component (B) is 0.05 meq/g or more and 6.5 meq/g or less.

5. The multilayer type composition according to any one of claims 1 to 4, wherein the component (C) includes at least one selected from the group consisting of an alkanolamine and sodium hydroxide.

6. The multilayer type composition according to any one of claims 1 to 5, wherein a mass ratio [(B)/(C)] of the component (B) to the component (C) is 0.1 or more and 20 or less.

7. The multilayer type composition according to any one of claims 1 to 6, wherein the component (D) contains 30% by mass or more of ethanol.

8. The multilayer type composition according to any one of claims 1 to 7, which is a hair cosmetic.

9. The multilayer type composition according to claim 8, wherein the hair cosmetic is a temporary hair dye.

10. Use of, as a hair cosmetic, a multilayer type composition comprising the following components (A) to (D):

    (A) an inorganic powder,
    (B) a cationic polymer,
    (C) an alkaline agent, and
    (D) a nonaqueous solvent.

11. The use according to claim 10, wherein the content of water in the multilayer type composition is 70% by mass or less.

**INTERNATIONAL SEARCH REPORT**

| International application No. |
|---|
| PCT/JP2020/015485 |

**A. CLASSIFICATION OF SUBJECT MATTER**
Int.Cl. A61K8/03(2006.01)i, A61K8/19(2006.01)i, A61K8/25(2006.01)i,
A61K8/34(2006.01)i, A61K8/41(2006.01)i, A61K8/72(2006.01)i,
A61K8/81(2006.01)i, A61Q5/00(2006.01)i, A61Q5/06(2006.01)i
FI: A61K8/03, A61K8/19, A61K8/25, A61K8/34, A61K8/41, A61K8/72,
A61Q5/00, A61Q5/06
According to International Patent Classification (IPC) or to both national classification and IPC

**B. FIELDS SEARCHED**

Minimum documentation searched (classification system followed by classification symbols)
Int.Cl. A61K8/03, A61K8/19, A61K8/25, A61K8/34, A61K8/41, A61K8/72,
A61K8/81, A61Q5/00, A61Q5/06

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y A | JP 11-79933 A (KOSE CORPORATION) 23.03.1999 (1999-03-23), claims, paragraphs [0001]-[0004], [0017], [0021], [0035] | 1-5, 7-11 6 |
| Y A | JP 2008-266241 A (HOYU CO., LTD.) 06.11.2008 (2008-11-06), paragraph [0039] | 1-5, 7-11 6 |
| A | JP 1-121209 A (KAO CORPORATION) 12.05.1989 (1989-05-12), claims | 1-11 |
| A | JP 2005-239626 A (KANEBO COSMETICS INC.) 08.09.2005 (2005-09-08), claims | 1-11 |

☐ Further documents are listed in the continuation of Box C.     ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09.06.2020 | 16.06.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

INTERNATIONAL SEARCH REPORT
Information on patent family members

International application No.

PCT/JP2020/015485

```
JP  11-79933 A       23.03.1999    (Family: none)

JP  2008-266241 A    06.11.2008    (Family: none)

JP  1-121209 A       12.05.1989    (Family: none)

JP  2005-239626 A    08.09.2005    (Family: none)
```

Form PCT/ISA/210 (patent family annex) (January 2015)

## REFERENCES CITED IN THE DESCRIPTION

**Patent documents cited in the description**

- JP 2016030725 A

- JP 1121209 A